# EUROPÄISCHE PATENTSCHRIFT

(11) **EP 0 820 987 B1**
(45) Veröffentlichungstag und Bekanntmachung des Hinweises auf die Patenterteilung: **06.11.2002**
(21) Anmeldenummer: 97111567.0
(22) Anmeldetag: 08.07.1997
(51) Int. Cl.: C07D 213/80, C07D 213/65

(54) **Verfahren zur Herstellung von Pyridin-2,3-dicarbonsäureestern**
Process for the preparation of pyridine-2,3-dicarboxylic acid esters
Procédé pour la préparation d'esters d'acide pyridine-2,3-dicarboxylique

(30) Priorität: 23.07.1996 CH 184096
(43) Veröffentlichungstag der Anmeldung: 28.01.1998
(73) Patentinhaber: Lonza AG, 3930 Visp (CH)
(72) Erfinder: Bessard, Ives, Dr., 3960 Sierre (Kanton Wallis) (CH); Stucky, Gerhard, Dr., 3902 Brig-Glis (Kanton Wallis) (CH); Roduit, Jean-Paul, Dr., 3979 Grône (Kanton Wallis) (CH)
(74) Vertreter: Winter, Brandl, Fürniss, Hübner, Röss, Kaiser, Polte Partnerschaft

(56) Entgegenhaltungen:
- EP-A- 0 461 401
- DE-A- 4 207 604
- US-A- 3 988 358
- US-A- 5 166 352
- US-A- 5 296 601
- CHEMICAL ABSTRACTS, vol. 125, no. 3, 15.Juli 1996 Columbus, Ohio, US; abstract no. 33323, XP002047196 & JP 08 073 403 A (NIHON NOHYAKU CO LTD) 19.März 1996

## Beschreibung

Die vorliegende Erfindung betrifft ein Verfahren zur Herstellung von Diestern substituierter Pyridin-2,3-dicarbonsäuren durch Umsetzung von 2,3-Dichlorpyridinen mit Kohlenmonoxid und einem Alkohol in Gegenwart eines Katalysators und einer Base. Die erfindungsgemäss herstellbaren Diester besitzen die allgemeine Formel

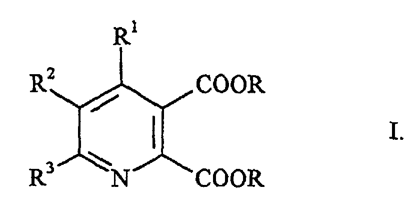

Hierin bedeuten:
R C₁₋₆-Alkyl, C₃₋₆-Cycloalkyl, oder gegebenenfalls mit C₁₋₆-Alkyl, halogeniertem C₁₋₆-Alkyl, C₁₋₆-Alkoxy, C₁₋₆-Alkylthio oder C₁₋₆-Alkansulfonyl substituiertes Aryl oder Aryl-C₁₋₆-alkyl,
R¹ bis R³ unabhängig voneinander Wasserstoff, C₁₋₆-Alkyl, fluoriertes C₁₋₆-Alkyl, C₁₋₆-Alkoxy, (C₁₋₆-Alkoxy)-C₁₋₆-alkyl oder (C₁₋₆-Alkoxy)carbonyl.

Zahlreiche Verbindungen dieser Struktur sind wichtige Herbizide bzw. Synthesebausteine für die Herstellung von Herbiziden (US-A 5 288 866). Die Synthese dieser bekannten Verbindungen geht üblicherweise von den entsprechenden Carbonsäuren oder Carbonsäurederivaten (Säurechloride, Nitrile) aus. Diese sind jedoch oft schwer zugänglich und deshalb teuer.
Ein weiteres Verfahren (US-A 5 296 601) zur Herstellung von Pyridin-2,3-dicarbonsäurediethylester geht von 2,3-Dichlorpyridin aus, welches mit Ethanol und Kohlenmonoxid in Gegenwart eines Palladiumkomplexes mit 1,4-Bis(diphenylphosphino)butan als Liganden und Natriumcarbonat als Base zum genannten Diester umgesetzt wird. Die Ausbeute ist jedoch so gering (<3%), dass eine technische Anwendung des Verfahrens völlig unwirtschaftlich wäre.

Aufgabe der vorliegenden Erfindung war daher, ein Verfahren zur Verfügung zu stellen, welches von leicht zugänglichen Edukten ausgeht und hohe Ausbeuten liefert.

Erfindungsgemäss wird diese Aufgabe durch das Verfahren nach Patentanspruch 1 gelöst. Es wurde gefunden, dass 2,3-Dichlorpyridine der allgemeinen Formel 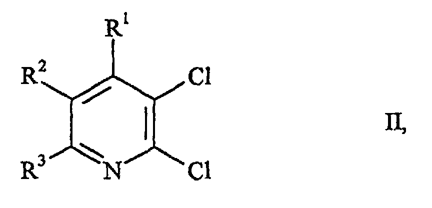 worin R¹ bis R³ die oben genannten Bedeutungen haben, mit Kohlenmonoxid und einem Alkohol der allgemeinen Formel

R-OH III,

worin R die oben genannte Bedeutung hat, in Gegenwart einer Base in guter Ausbeute direkt zu den gewünschten Produkten (I) reagieren, wenn als Katalysator ein Komplex von Palladium mit einem Diphosphin der allgemeinen Formel

R⁴R⁵P―Q―PR⁶R⁷ IV,

worin R⁴ bis R⁷ unabhängig voneinander gegebenenfalls substituiertes Phenyl, C₁₋₆-Alkyl oder C₃₋₆-Cycloalkyl bedeuten und Q eine 1,1'-Ferrocendiylgruppe oder eine Gruppe der Formel -[CH₂]_{*n*}-, worin *n* 3 oder 4 ist, bedeutet, anwesend ist und als Base eine schwache Base aus der Gruppe der Alkali- oder Erdalkalisalze von niedrigen Carbonsäuren und der Alkali- oder Erdalkalihydrogencarbonate eingesetzt wird.

Unter C₁₋₆-Alkyl sind hier und im folgenden alle linearen oder verzweigten primären, sekundären oder tertiären Alkylgruppen mit bis zu 6 Kohlenstoffatomen zu verstehen. Entsprechend sind unter C₁₋₆-Alkoxy und (C₁₋₆-Alkoxy)carbonyl die aus C₁₋₆-Alkyl und Sauerstoff bzw. Sauerstoff und Carbonyl zusammengesetzten Ether- und Esterfunktionen zu verstehen und analog dazu unter (C₁₋₆-Alkoxy)-C₁₋₆-alkyl die durch Austausch eines Wasserstoffatoms in C₁₋₆-Alkyl gegen C₁₋₆-Alkoxy gebildeten Alkoxyalkylgruppen, also beispielsweise Methoxymethyl oder Ethoxymethyl.
Unter Aryl sind hier und im folgenden insbesondere mono- oder polycyclische Systeme wie beispielsweise Phenyl, Naphthyl, Biphenylyl oder Anthracenyl zu verstehen. Diese können einen oder mehrere gleiche oder verschiedene Substituenten tragen, beispielsweise niedrige Alkylgruppen wie Methyl, halogenierte Alkylgruppen wie Trifluormethyl, niedrige Alkoxygruppen wie Methoxy, oder niedrige Alkylthio- (Alkansulfanyl-) oder Alkansulfonylgruppen wie Methylthio oder Ethansulfonyl. Unter substituiertem Phenyl sind insbesondere Gruppen wie Fluorphenyl, Methoxyphenyl, Tolyl oder Trifluormethylphenyl zu verstehen, wobei sich die Substituenten vorzugsweise in der *para*-Position befinden. Entsprechend sind unter Arylalkyl die aus niedrigen Alkylgruppen, insbesondere C₁₋₆-Alkyl, durch Austausch eines Wasserstoffatoms gegen eine der vorstehend definierten Arylgruppen gebildeten Gruppen zu verstehen, also beispielsweise Benzyl oder Phenylethyl.

Die als Ausgangsmaterial dienenden 2,3-Dichlorpyridine (II) sind teilweise bekannte Verbindungen oder können analog zu bekannten Verbindungen hergestellt werden. (Siehe z.B. L. A. Paquette, *Encyclopedia of Reagents for Organic Synthesis,* John Wiley & Sons, New York, **1995** und CH-A 664 754.)

Vorzugsweise werden nach dem erfindungsgemässen Verfahren Methyl- oder Ethylester hergestellt (R = Me, Et), indem als Alkohol (III) Methanol oder Ethanol eingesetzt wird.

Ebenfalls bevorzugt ist die Herstellung von Pyridin-2,3-dicarbonsäureestern (I), die in den Positionen 4 und 6 des Pyridinrings unsubstituiert sind (R¹ = R³ = H).

Besonders bevorzugt ist die Herstellung von Pyridin-2,3-dicarbonsäureestern (I), die in der Position 5 des Pyridinrings (R²) Wasserstoff, eine (C₁₋₄-Alkoxy)carbonylgruppe oder eine (C₁₋₄)-Alkoxy)methylgruppe tragen.

Der katalytisch wirksame Palladium-Diphosphin-Komplex wird vorteilhaft *in situ* gebildet, indem Palladium in fein verteilter elementarer Form (z. B. Palladium auf Aktivkohle), ein Pd(II) Salz (z. B. das Chlorid oder das Acetat), oder ein geeigneter Pd(II)-Komplex (z. B. Dichloro-bis(triphenylphosphin)palladium(II)) mit dem Diphosphin zur Reaktion gebracht wird. Besonders bevorzugt sind Palladium(II)acetat und Dichloro-bis(triphenylphosphin)palladium(II). Das Palladium wird vorzugsweise in einer Menge von 0,02 bis 5 Mol-% Pd(II) oder 0,5 bis 5 Mol-% Pd(0) (als Pd/C), jeweils bezogen auf die Halogenverbindung (II), eingesetzt. Das Diphosphin wird vorteilhaft im Überschuss (bezogen auf Pd) eingesetzt, vorzugsweise in einer Menge von 0,2 bis 10 Mol-%, ebenfalls bezogen auf die Halogenverbindung (II).

Der Alkohol (III) kann auch gleichzeitig als Lösungsmittel dienen. Gegebenenfalls kann ein zusätzliches Lösungsmittel eingesetzt werden. Als zusätzliche Lösungsmittel kommen sowohl relativ unpolare, wie beispielsweise Toluol oder Xylol, als auch polare wie beispielsweise Acetonitril, Tetrahydrofuran oder *N,N*-Dimethylacetamid in Frage.

Als Base wird vorzugsweise ein Alkaliacetat eingesetzt. Besonders bevorzugt sind Natrium- und Kaliumacetat.

Die Reaktionstemperatur liegt vorzugsweise bei 80 bis 250 °C.

Der Kohlenmonoxiddruck liegt vorzugsweise bei 1 bis 50 bar.

Die Reaktionsdauer hängt unter anderem von der Temperatur, der Reaktivität der eingesetzten Verbindungen und den Konzentrationsverhältnissen ab, sie liegt typischerweise im Bereich von einigen Stunden. Da die beiden Chloratome des Dichlorpyridins (II) nacheinander substituiert werden und bei übermässig langer Reaktionsdauer unter Umständen Folgereaktionen eintreten, wird der Reaktionsverlauf vorteilhaft mit einer geeigneten Analysenmethode (z. B. GC) überwacht und die Reaktion nach Erreichen der maximalen Produktkonzentration abgebrochen.

Die folgenden Beispiele verdeutlichen die Durchführung des erfindungsgemässen Verfahrens.

### Beispiel 1

### Pyridin-2,3-dicarbonsäurediethylester

### (I, R = Et, R¹ = R² = R³ = H)

In einem Metallautoklaven wurden 1,52 g (10 mmol) 2,3-Dichlorpyridin (Fluka), 166 mg (0,3 mmol) 1,1'-Bis(diphenylphosphino)ferrocen, 22,4 mg (0,1 mmol) Palladium(II)acetat, 1,72 g (21 mmol) Natriumacetat und 25 ml Ethanol vorgelegt. Der Autoklav wurde mehrmals mit Kohlenmonoxid gespült, dann wurde der Kohlenmonoxiddruck auf 15 bar erhöht und das Reaktionsgemisch 2,5 h auf 135 °C (Innentemperatur) erhitzt. Anschliessend wurde das Reaktionsgemisch im Vakuum eingeengt und der Rückstand an Kieselgel 60 mit Hexan/Ethylacetat (3:1) chromatographiert.
- Ausbeute:: 0,945 g (85%) farbloses Öl
- ¹H NMR (CDCl₃) δ =: 8,75 (d, 1H); 8,19 (d, 1H); 7,47 (dd, 1H); 4,48 (q, 2H); 4,39 (q, 2H); 1,44 (t, 3H); 1,39 (t, 3H).
- MS (*m*/*z*):: 223 (M⁺), 179; 150; 122; 107; 79.

### Beispiel 2

### Pyridin-2,3,5-tricarbonsäuretrimethylester

### (I, R = Me, R¹ = R³ = H, R² = COOMe)

Analog zu Beispiel 1 wurden 2,06 g (10 mmol) 5,6 -Dichlornikotinsäuremethylester (II, R¹ = R³ = H, R² = COOMe), 128 mg (0,3 mmol) 1,4-Bis(diphenylphosphino)butan, 14,0 mg (20 µmol) Dichlorobis(triphenylphosphin)palladium(II) und 2,46 g (30 mmol) Natriumacetat in 25 ml Methanol bei 155 °C (Badtemperatur) und 15 bar CO-Druck 6 h umgesetzt. Eine GC-Analyse des Reaktionsgemischs ergab 77% Ausbeute bei 100% Umsatz.
- Isolierte Ausbeute:: 800 mg (50%) gelbes Öl
- ¹H NMR (CDCl₃) δ =: 9,31 (s, 1H); 8,78 (s, 1H); 4,01 (s, 3H); 4,00 (s, 3H); 3,97 (s, 3H).
- MS (*m*/*z*):: 253 (M⁺); 223; 222; 195; 194; 165; 137.

### Beispiel 3

### 2,3-Dichlor-5-(methoxymethyl)pyridin

### (II, R¹ = R³ = H, R² = CH₂OMe)

Unter Argon wurden 4,14 g (23 mmol) Natriummethanolat (30%ige Lösung in Methanol) bei Raumtemperatur innerhalb von 5 min zu einer Lösung von 4,11 g (20,9 mmol) 2,3-Dichlor-5-(chlormethyl)pyridin (hergestellt aus 2,3-Dichlor-5-(hydroxymethyl)pyridin durch Umsetzung mit Thionylchlorid) in 40 ml Methanol getropft. Anschliessend wurde das Reaktionsgemisch 3 h auf 60 °C erwärmt. Nach dem Ende der Reaktion wurde das Lösungsmittel abdestilliert, der Rückstand mit 100 ml Wasser versetzt und mit Dichlormethan (3 × 75 ml) extrahiert. Die organische Phase wurde über Magnesiumsulfat getrocknet und eingedampft.
- Ausbeute:: 4,06 g (90,4%) gelbes Öl, Gehalt (GC) 90,8%
Zur Analyse wurde das Produkt mit Hexan/Ethylacetat (3:1) an Kieselgel chromatographiert.
- ¹H NMR (CDCl₃) δ =: 8,25 (s, 1H); 7,78 (s, 1H); 4,46 (s, 2H); 3,43 (s, 3H).
- MS (*m*/*z*):: 192 (M⁺); 176; 161; 148; 124; 112.

### Beispiel 4

### 5-(Methoxymethyl)pyridin-2,3-dicarbonsäuredimethylester

### (I, R = Me, R¹ = R³ = H, R² = CH₂OMₑ)

Analog zu Beispiel 1 wurden 1,92 g (10 mmol) 2,3-Dichlor-5-(methoxymethyl)pyridin (hergestellt nach Beispiel 3), 166 mg (0,3 mmol) 1,1'-Bis(diphenylphosphino)ferrocen, 14 mg (20 µmol) Dichlorobis(triphenylphosphin)palladium(II) und 2,46 g (30 mmol) Natriumacetat in 25 ml Methanol bei 160 °C (Badtemperatur) und 15 bar CO-Druck 24 h umgesetzt. Eine GC-Analyse des Reaktionsgemischs ergab 83% Ausbeute bei 100% Umsatz.
- Isolierte Ausbeute:: 500 mg (41%) gelbes Öl, Gehalt (GC) 97%
- ¹H NMR (CDCl₃) δ =: 8,70 (s, 1H); 8,15 (s, 1H); 4,57 (s, 2H); 4,00 (s, 3H); 3,92 (s, 3H); 3,43 (s, 3H).
- MS (*m*/*z*):: 239 (M⁺); 209; 181; 151; 123.

### Beispiel 5

### 5-(Methoxymethyl)pyridin-2,3-dicarbonsäuredimethylester

### (I, R = Me, R¹ = R³ = H, R² = CH₂OMe)

Es wurde verfahren wie in Beispiel 4 beschrieben, jedoch wurde die Reaktionstemperatur auf 180 °C (Badtemperatur) erhöht und die Reaktionsdauer auf 4 h reduziert. Eine GC-Analyse des Reaktionsgemischs ergab 79% Ausbeute bei 100% Umsatz. Isolierte Ausbeute: 750 mg (58%) gelbes Öl, Gehalt (GC) 92,4%

### Beispiel 6

### 5-(Methoxymethyl)pyridin-2,3-dicarbonsäuredimethylester

### (I, R = Me, R¹ = R³ = H, R² = CH₂OMe)

Es wurde verfahren wie in Beispiel 4 beschrieben, jedoch wurde anstelle von Dichlorobis(triphenylphosphin)Palladium((II) 4,4 mg (20 µmol) Palladium(II)acetat eingesetzt. Die Reaktionstemperatur betrug 160-170 °C (Badtemperatur), die Reaktionsdauer 5 h. Eine GC-Analyse des Reaktionsgemischs ergab 90% Ausbeute bei 100% Umsatz,
- Isolierte Ausbeute:: 740 mg (61%) gelbes Öl, Gehalt (GC) 100%

### Beispiel 7

### 5-(Methoxymethyl)pyridin-2,3-dicarbonsäuredimethylester

### (I, R = Me, R¹ = R³ = H, R² = CH₂OMₑ)

Es wurde verfahren wie in Beispiel 6 beschrieben, jedoch wurde anstelle von Natriumacetat 2,94 g (30 mmol) Kaliumacetat eingesetzt. Die Reaktionstemperatur betrug 170 °C (Badtemperatur), die Reaktionsdauer 3 h. Eine GC-Analyse des Reaktionsgemischs ergab 90% Ausbeute bei 100% Umsatz.

### Beispiel 8

### 5-(Methoxymethyl)pyridin-2,3-dicarbonsäurediethylester

### (I, R = Et, R¹ = R³ = H, R² = CH₂OMe)

Analog zu Beispiel 4 wurden 1,92 g (10 mmol) 2,3-Dichlor-5-(methoxymethyl)pyridin, 166 mg (0,3 mmol) 1,1'-Bis(diphenylphosphino)ferrocen, 14,0 mg (20 µmol) Dichlorobis(triphenylphosphin)palladium(und 2,46 g (30 mmol) Natriumacetat in 25 ml Ethanol bei 175 °C (Badtemperatur) und 15 bar CO-Druck 4 h umgesetzt. Eine GC-Analyse des Reaktionsgemischs ergab 79% Ausbeute bei 100% Umsatz.
- Isolierte Ausbeute:: 920 mg (67%) gelbes Öl, Gehalt (GC) 98,1%
- ¹HNMR (CDCl₃) δ =: 8,70 (s, 1H); 8,15 (s, 1H); 4,55 (s, 2H); 4,46 (q, 2H); 4,40 (q, 2H); 3,44 (s, 3H); 1,42 (t, 3H); 1,38 (t, 3H).
- MS (*m*/*z*):: 267 (M⁺); 236; 223; 194; 151; 123.

### Beispiel 9

### 5-(Methoxymethyl)pyridin-2,3-dicarbonsäurediethylester

### (I, R = Et, R¹ = R³ = H, R² = CH₂OMe)

Es wurde verfahren wie in Beispiel 8 beschrieben, jedoch wurde anstelle von Dichlorobis(triphenylphosphin)palladium(II) 4,4 mg (20 µmol) Palladium(II)acetat eingesetzt und die Natriumacetatmenge auf 1,72 g (21 mmol) reduziert. Die Reaktionstemperatur betrug 155 °C (Innentemperatur), die Reaktionsdauer 2 h. Eine GC-Analyse des Reaktionsgemischs ergab 88% Ausbeute bei 100% Umsatz.
- Isolierte Ausbeute:: 950 mg (67%) gelbes Öl, Gehalt (GC) 94%

### Beispiel 10

### 5-(Methoxymethyl)pyridin-2,3-dicarbonsäurediethylester

### (I, R = Et, R¹ = R³ = H, R² = CH₂OMₑ)

Es wurde verfahren wie in Beispiel 9 beschrieben, jedoch betrug die Reaktionstemperatur 145 °C (Innentemperatur) und die Reaktionsdauer 5 h. Eine GC-Analyse des Reaktionsgemischs ergab 95% Ausbeute bei 100% Umsatz.
- Isolierte Ausbeute:: 1100 mg (78,7%) gelbes Öl, Gehalt (GC) 95,6%

### Beispiel 11

### 5-(Methoxymethyl)pyridin-2,3-dicarbonsäurediethylester

### (I, R = Et, R¹ = R³ = H, R² = CH₂OMe)

Es wurde verfahren wie in Beispiel 9 beschrieben, jedoch wurden 11 mg (50 µmol) Palladium(II)acetat eingesetzt. Eine GC-Analyse des Reaktionsgemischs ergab 98% Ausbeute bei 100% Umsatz.
- Isolierte Ausbeute:: 1260 mg (90%) gelbes Öl, Gehalt (GC) 95,4%

## Patentansprüche

1. Verfahren zur Herstellung von Pyridin-2,3-dicarbonsäureestern der allgemeinen Formel 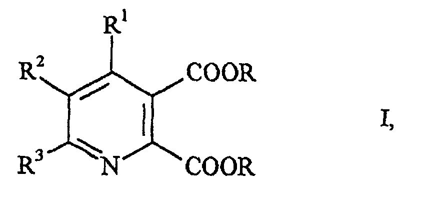 worin R C₁₋₆-Alkyl, C₃₋₆-Cycloalkyl, oder gegebenenfalls mit C₁₋₆-Alkyl, halogeniertem C₁₋₆-Alkyl, C₁₋₆-Alkoxy, C₁₋₆-Alkylthio oder C₁₋₆-Alkansulfonyl substituiertes Aryl oder Aryl-C₁₋₆-alkyl ist und R¹ bis R³ unabhängig voneinander Wasserstoff, C₁₋₆-Alkyl, fluoriertes C₁₋₆-Alkyl,C₁₋₆-Alkoxy, (C₁₋₆Alkoxy)-C₁₋₆-alkyl oder (C₁₋₆-Alkoxy)carbonyl bedeuten, worin ein 2,3-Dichlorpyridin der allgemeinen Formel 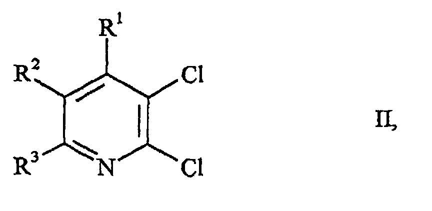 worin R¹ bis R³ die oben genannten Bedeutungen haben, mit Kohlenmonoxid und einem Alkohol
R-OH III,
worin R die oben genannte Bedeutung hat, in Gegenwart eines katalytisch aktiven Komplexes von Palladium mit einem Diphosphin der allgemeinen Formel
R⁴R⁵P―Q―PR⁶R⁷ IV,
worin R⁴ bis R⁷ unabhängig voneinander Phenyl, substituiertes Phenyl, C₁₋₆-Alkyl oder C₃₋₆-Cycloalkyl bedeuten, und Q eine 1,1'-Ferrocendiylgruppe oder eine Gruppe der Formel -[CH₂]_{*n*}-, worin *n* 3 oder 4 ist, bedeutet, und einer Base zur Reaktion gebracht wird, **dadurch gekennzeichnet, daß** die Base ausgewählt ist aus der Gruppe bestehend aus den Alkali- und Erdalkalisalzen von niedrigen Carbonsäuren und den Alkali- und Erdalkalihydrogencarbonaten.

2. Verfahren nach Anspruch 1, **dadurch gekennzeichnet, dass** R Methyl oder Ethyl ist.

3. Verfahren nach Anspruch 1 oder 2, **dadurch gekennzeichnet, dass** R¹ und R³ Wasserstoff sind.

4. Verfahren nach einem der Ansprüche 1 bis 3, **dadurch gekennzeichnet, dass** R² Wasserstoff, (C₁₋₄-Alkoxy)carbonyl oder (C₁₋₄-Alkoxy)methyl ist.

5. Verfahren nach einem der Ansprüche 1 bis 4, **dadurch gekennzeichnet, dass** der katalytisch aktive Palladiumkomplex *in situ* aus dem Diphosphin (IV) und Palladium(II)acetat oder Dichloro-bis(triphenylphosphin)palladium(II) gebildet wird.

6. Verfahren nach einem der Ansprüche 1 bis 5, **dadurch gekennzeichnet, dass** als Base ein Alkaliacetat, vorzugsweise Natriumacetat oder Kaliumacetat, eingesetzt wird.

## Claims

1. Process for producing pyridine-2,3-dicarboxylic acid esters having the general formula 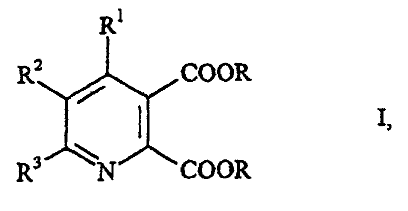 wherein R is C₁₋₆ alkyl, C₃₋₆ cycloalkyl or aryl or aryl C₁₋₆ alkyl optionally substituted with C₁₋₆ alkyl, halogenated C₁₋₆ alkyl, C₁₋₆ alkoxy, C₁₋₆ alkyl thio or C₁₋₆ alkane sulfonyl and R¹ to R³ mutually independently denote hydrogen, C₁₋₆ alkyl, fluorinated C₁₋₆ alkyl, C₁₋₆ alkoxy, (C₁₋₆ alkoxy) C₁₋₆ alkyl or (C₁₋₆ alkoxy) carbonyl, wherein a 2,3-dichloropyridine having the general formula 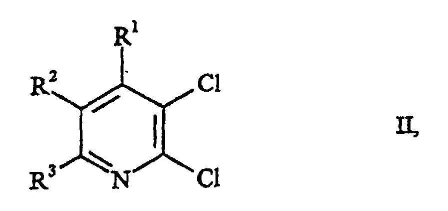 wherein R¹ to R³ have the meanings cited above, is reacted with carbon monoxide and an alcohol
R-OH III,
wherein R has the meaning cited above, in the presence of a catalytically active complex of palladium with a diphosphine having the general formula
R⁴R⁵P―PR⁶R⁷ IV,
wherein R⁴ to R⁷ mutually independently denote phenyl, substituted phenyl, C₁₋₆ alkyl or C₃₋₆ cycloalkyl, and Q denotes a 1,1'-ferrocene diyl group or a group having the formula -[CH₂]ₙ-, wherein *n* is 3 or 4, and a base, **characterised in that** the base is selected from the group consisting of the alkali and alkaline-earth salts of low carboxylic acids and the alkali and alkaline-earth hydrogen carbonates.

2. Process according to claim 1, **characterised in that** R is methyl or ethyl.

3. Process according to claim 1 or 2, **characterised in that** R¹ and R³ are hydrogen.

4. Process according to one of claims 1 to 3, **characterised in that** R² is hydrogen, (C₁₋₄ alkoxy) carbonyl or (C₁₋₄ alkoxy) methyl.

5. Process according to one of claims 1 to 4, **characterised in that** the catalytically active palladium complex is formed *in situ* from the diphosphine (IV) and palladium(II) acetate or dichloro-bis(triphenyl phosphine) palladium(II).

6. Process according to one of claims 1 to 5, **characterised in that** an alkali acetate, preferably sodium acetate or potassium acetate, is used as a base.

## Revendications

1. Procédé pour la préparation d'esters d'acide pyridine-2,3-dicarboxylique de la formule générale : 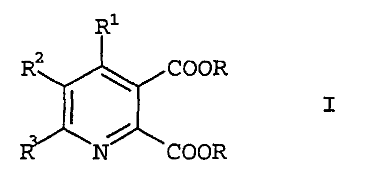 dans laquelle R est un C₁₋₆-alkyle, un C₃₋₆-cycloalkyle ou, le cas échéant, un C₁₋₆-alkyle halogéné avec un C₁₋₆-alkyle, un C₁₋₆-alcoxy, un C₁₋₆-alkylthio ou un aryle substitué par un C₁₋₆-alcanesulfonyle ou un aryle-C₁₋₆-alkyle, et R¹ à R³, représentent indépendamment l'un de l'autre, un atome d'hydrogène, un C₁₋₆-alkyle, un C₁₋₆-alkyle fluoré, un C₁₋₆-alcoxy, un (C₁₋₆-alcoxy) -C₁₋₆-alkyle ou un (C₁₋₆-alcoxy)carbonyle, dans lequel une dichlopyridine-2,3 de la formule générale : 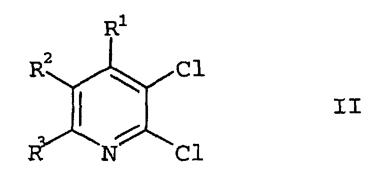 dans laquelle R¹ à R³ ont la signification indiquée ci-dessus, est mis à réagir avec un monoxyde de carbone et un alcool :
R-OH III,
dans lequel R a la signification indiquée ci-dessus, en présence d'un complexe catalytiquement actif de palladium avec une diphosphine de la formule générale
R⁴R⁵P―Q―pR⁶R⁷ IV,
où R⁴ à R⁷, représentent indépendamment l'un de l'autre, un phényle, un phényle substitué, un C₁₋₆-alkyle ou un C₃₋₆-cycloalkyle et Q représente un groupe ferrocendiyle-1,1' ou un groupe de la formule -[CH₂]_{*n*}-, où *n* vaut 3 ou 4, ainsi qu'avec une base, **caractérisé par le fait que**, la base a été choisie dans le groupe constitué par les sels alcalins ou alcalino-terreux d'acides carbonés inférieurs et par les hydrocarbonates alcalins ou alcalino-terreux.

2. Procédé suivant la revendication 1, **caractérisé par le fait que** R est le méthyle ou l'éthyle.

3. Procédé suivant la revendication 1 ou 2, **caractérisé par le fait que** R¹ et R³ sont un atome d'hydrogène.

4. Procédé suivant une des revendications de 1 à 3, **caractérisé par le fait que** R² est un atome d'hydrogène, un (C₁₋₄-alcoxy)carbonyle ou un (C₁₋₆-alcoxy)méthyle.

5. Procédé suivant une des revendications de 1 à 4, **caractérisé par le fait que** le complexe de palladium catalytiquement actif se forme *in situ* à partir de la diphosphine (IV) et de l'acétate de paladium(II) ou du dichloro-bis(triphénylephosphine) palladium(II).

6. Procédé suivant une des revendications de 1 à 5, **caractérisé par le fait que** l'on utilise comme base un acétate alcalin, de préférence un acétate de sodium ou un acétate de potassium.
